# EUROPEAN PATENT SPECIFICATION

(11) **EP 4 089 040 B1**
(45) Date of publication and mention of the grant of the patent: **11.11.2026**
(21) Application number: 21173193.0
(22) Date of filing: 11.05.2021
(51) Int. Cl.: B65G 54/02

(54) **ADAPTIVE CARRIER UNIT AND TRANSFER SYSTEM**
ADAPTIVE TRÄGEREINHEIT UND TRANSFERSYSTEM
UNITÉ DE SUPPORT ADAPTATIF ET SYSTÈME DE TRANSFERT

(43) Date of publication of application: 16.11.2022
(73) Proprietor: Fameccanica.Data S.p.A., 66020 San Giovanni Teatino (CH) (IT)
(72) Inventor: SABLONE, Gabriele, I-66020 San Giovanni Teatino (Chieti) (IT); ROSSETTI, Massimiliano, I-66020 San Giovanni Teatino (Chieti) (IT); DI SABATINO, Carlo, I-66020 San Giovanni Teatino (Chieti) (IT); DELLI ROCIOLI, Marco, I-66020 San Giovanni Teatino (Chieti) (IT)
(74) Representative: Buzzi, Notaro & Antonielli d'Oulx S.p.A.

(56) References cited:
- EP-A1- 2 741 979
- EP-A1- 3 744 649
- WO-A1-2020/030836
- US-A1- 2015 136 564
- US-A1- 2016 176 659
- US-A1- 2019 367 297

## Description

### Field of the invention

The present invention relates to transfer systems, particularly transfer systems for products such as diapers or sanitary napkins which are intended to be packaged as stacks of products, and transferred in stacked or packed fashion as well. Such transfer systems are known, for instance, from US 2016/0176659 A1.

### Prior art

US 2016/176659 A1 discloses a carrier unit according to the preamble of claim 1.

Transfer systems for the above mentioned products generally involve a transfer path featuring one or more bends or changes of direction. This path is conveniently negotiated by means of so-called "flexible carriers" such as ACOPOSTrak^{®} carriers, which allow negotiation of complex paths through multiple, selectable, tracks and related junctions. Embedded in the tracks are driving means such as, for instance, linear motors (stator units).

Flexible carriers of this type, as well known in the art, are configured to engage a driving track on either side (left/right) thereof. Change of track is usually accommodated by changing the engagement side of the carrier, whereby a carrier moving - for instance - along a first track with left side out, right side in (facing the track) may accomplish track change to a second track at a junction by simply switching the engagement side from left to right, hence moving along the second track left side in, right side out.

While allowing exceptional flexibility, this feature also comes with a first major limit: any accessory, implement, end effector, payload, etc. mounted on the carrier must be positioned on top of the carrier itself to avoid interfering at the sides of the carrier, which would prevent engagement with a driving track.

A second major limit of such flexible carriers is the need to avoid disruption of the engagement with the driving track - typically achieved thanks to weak magnetic attraction - by inertial forces, which generally results in limitations to the process speed of the carriers and/or in the need for use of twin (split) grippers, i.e. pair-wise arranged grippers rigidly mounted on pairs of carriers.

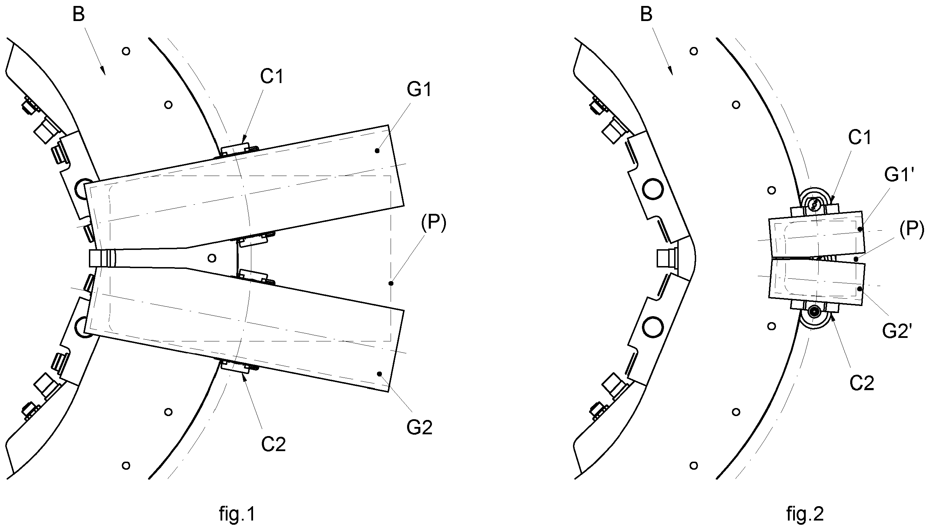

The first mentioned solution (reduction of process speeds) is largely unacceptable as it affects further processing stages. The second mentioned solution (pair-wise grippers) turns out to be effective only when negotiating rectilinear portions of the tracks, as negotiating a bend results in a change in the overall geometry of the gripping socket defined by the two grippers on the carriers. Reference is made, in this regard, to figures 1 and 2 which show a prior art transfer system featuring flexible carriers, and particularly a bend portion B of the driving track. Engaged on the driving track are a first and a second carriers C1, C2 each featuring a gripper G1, G2 (figure 1, large products) or G1', G2' (figure 2, small products) mounted thereon and located at the bend portion B.

Figures 1 and 2 clearly show that the twin grippers require adaptation based on the product dimension (grippers G1, G2 are used for large products, while grippers G1', G2' are used for small products), but also - most notably - that the interference conditions with the products varies through the bend B due to dependency thereof from the radius of curvature thereof, and based on the product dimension (the rectangular outline referred to as (P) in figures 1 and 2 is representative of the product dimension - when undeformed). More specifically, the smaller the radius, the larger the variation in the interference conditions, and the larger the product (and the grippers), the larger the variation of interference conditions.

This is clearly unacceptable, as it may ultimately result in the need for reducing processing speeds due to varied interference conditions, which may turn out to be insufficient to counter centrifugal forces when negotiating the bend.

### Object of the invention

The object of the invention is to overcome the technical problems discussed in the foregoing.

Specifically, the object of the invention is to provide a transfer system which is easily adaptable to products of different dimensions, and which is essentially unaffected by bends or changes of directions in the path to be negotiated as far as the interference conditions with the products is concerned.

### Summary of the invention

The object of the invention is achieved by a carrier unit and a transfer system having the features forming the subject of the appended claims, which form an integral part of the technical disclosure herein provided in relation to the invention.

### Brief description of the figures

Further features and advantages of the invention will become apparent from the following description with reference to the annexed figures, provided purely by way of non limiting example, wherein:
- Figures 1 and 2 are representative of prior art transfer systems,
- Figure 3 is a front, elevational view of a transfer system with a carrier unit not according to the invention, while figure 4 is a perspective view according to pointer IV in figure 3,
- Figure 5 is a sectional view according to the plane V-V in figure 4,
- Figure 6 is a sectional view according to the plane VI-VI in figure 3,
- Figure 7 is a sectional view according to the plane VII-VII in figure 3,
- Figure 8 represents an input station to the transfer system, as well as a view of the transfer system itself,
- Figure 9 corresponds to a plan view of the transfer system and corresponds to the pointer IX in figure 8, while the pointer VIII in figure 9 identifies the position of the input station of figure 8 relative to the transfer system,
- Figures 10 and 11 are, respectively, a plan and an elevational view of a transfer system with a carrier unit not according to the invention, and
- Figure 12 is a plan view of a carrier unit according to the invention.

### Detailed description

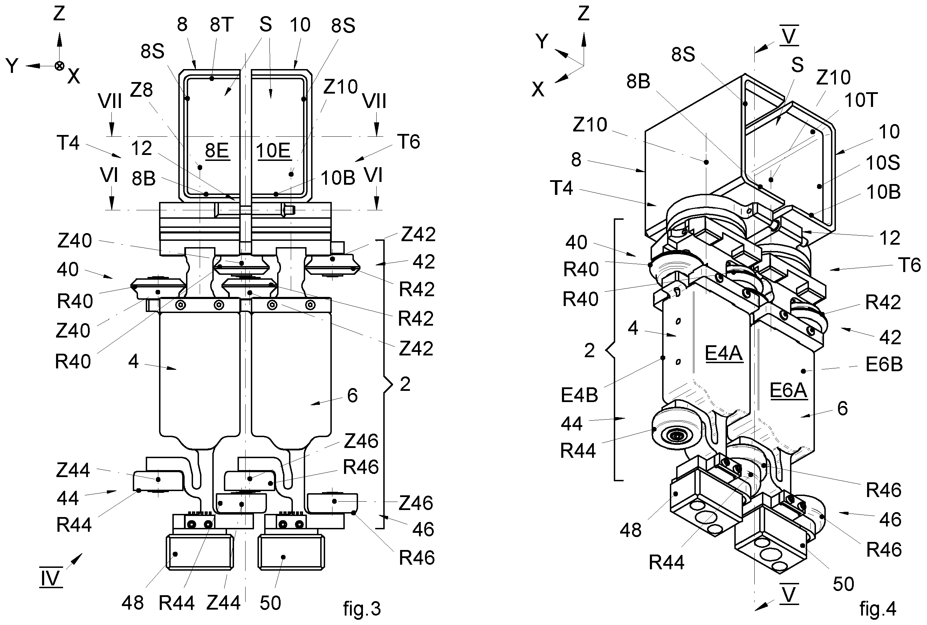

Reference number 1 in figures 3 and 4 designates as a whole a carrier for a transfer system comprising a transfer path.

The carrier unit 1 comprises a pair 2 of carriers (or, better put, a paired arrangement of carriers) comprising a first carrier 4 and a second carrier 4, both individually engageable on a portion of the transfer path and configured for negotiating the transfer path itself. The carriers 4 and 6 are preferably embodied as flexible, electromagnetic driven carriers such as those known under the commercial name ACOPOSTrak^{®}. At any rate, the carriers 4 and 6 may be embodied by other, more conventional, carriers such as mechanically engageable and driven carriers.

The first carrier 4 is coupled to a first gripper 8 configured for cooperating with a second gripper 10 coupled to the second carrier 6 to define a socket S for holding one or more products to be carried through at least part of the transfer path.

More in detail, each of the grippers 8, 10 is provided as an essentially box-shaped member each including:
- a side wall 8S, 10S
- a top wall 8T, 10T
- a bottom wall 8B, 10B
- an end wall 8E, 10E
whereby when the grippers 8, 10 are set alongside one another the side walls 8S, 10S face one another and the end walls 8E, 10E, as well as the top and bottom walls 8T, 10T and 8B, 10B are substantially arranged coplanar to one another to define the socket S.

In general, the socket S has a socket geometry determined by a relative position of the first gripper 8 with respect to the second gripper 10. The socket geometry is susceptible of variation as a function of the relative position of the first and second gripper 8, 10 with respect to one another and the relative position of each gripper 8, 10 with respect to the carrier 4, 6.

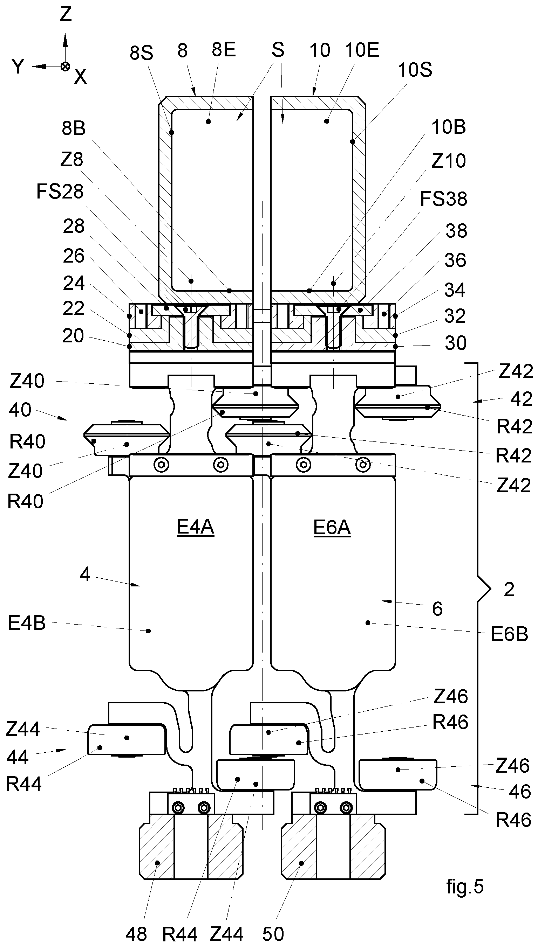

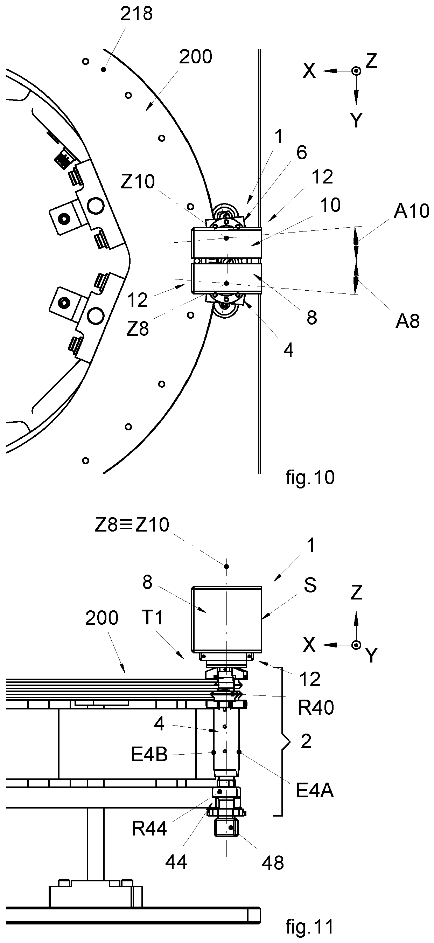

More specifically, (directions mentioned in the following are referred to a right hand reference system X-Y-Z represented in each of figures 3-11):
- the first gripper 8 is rotatably coupled to the first carrier 4 around a respective first axis of rotation Z8, whereby the relative position of the first gripper 8 with respect to the first carrier 4 is defined by a first angle A8 (figure 10) between the first gripper 8 and the first carrier 4 around the first axis of rotation Z8,
- the second gripper 10 is rotatably coupled to the second carrier 6 around a respective second axis of rotation Z10, whereby the relative position of the second gripper 10 with respect to the second carrier 6 is defined by a second angle A10 (figure 10) between the second gripper 10 and the second carrier 6 around the second axis of rotation Z10,
- each carrier unit 1 comprises an angle control device 12 (which will be extensively detailed in the following) configured for adjusting the first angle A8 and the second angle A10 to maintain a predetermined socket geometry upon negotiation of a transfer path by the carrier unit 1.

With reference to figures 3-7, in preferred embodiments the angle control device 12 comprises a sliding coupling between the first gripper 8 and the second gripper 10, the sliding coupling comprising a first coupling element 16 fixed to the first gripper 8 and a second coupling element 18 fixed to the second gripper 10. The first coupling element 16 is being configured to slide with respect to the second coupling element 18 in a direction Y16 transverse to the first axis Z8 and to the second axis Z10 and extending across the first axis and the second axis Z8, Z10. The number of pair of coupling elements may vary based on specific requirements, for instance for achieving better and more stable motion performances and/or dynamic performances.

In the preferred embodiment shown in the figures, the angle control device 12 for instance comprises two pairs of coupling elements 16, 18 (references A and B in parentheses reflect this) wherein:
- a first pin 16(A) and a second pin 16(B) fixed to the first gripper 8 on opposite sides of the axis Z8, the first pin and the second pin having respective longitudinal axes Y16 parallel to one another and transverse to the first axis Z8,
- a first hub 18(A) and a second hub 18(B) on opposite sides of the axis Z10 and provided on said second gripper 10, the first pin 16(A) being slidably coupled to the first hub 18(A) and the second pin 16(B) being slidably coupled to the second hub 18(B).

Each of the first pin 16(A) and the second pin 16(B) is cylindrical in shape, and is coaxially coupled, respectively, to the first hub 18(A) and the second hub 18(B) which are also cylindrical in shape. Preferably, a low-friction lining is provided inside the hub 18(A) and 18(B) to reduce friction upon sliding of the pins 16(A) and 16(B) through the hubs 18(A) and 18 (B) .

In alternative embodiments each of the first and second pins 16(A) and 16(B) is prismatic in shape and is shape coupled, respectively, to the first hub 18(A) and the second hub 18(B) to define a first prismatic guide and a second prismatic guide. From the stand point of available degrees of freedom in the angle control device 12, the use of cylindrical pins or prismatic pins has the same result, i.e. the sole available degree of freedom is the axial translation along direction Y16. The shape of the pins may be chosen, for instance, for structural reasons, constructional or assembly reasons, and so on.

With reference, again, to figures 3 to 7, in preferred embodiments the first carrier 4 and the second carrier 6 each include a turntable assembly (T1, T2 respectively) by which rotatable coupling of the respective gripper 8, 10 to the carrier 4, 6 is achieved.

In detail, the turntable assembly T1 of the carrier 4 comprises a base plate 20 with a protruding, internally threaded hub portion, a thrust plate 22 fitted onto the hub of the base plate 20 and having itself a hub portion fitted onto the hub portion of the base plate 20 and lying flush with the protruding hub portion of the base plate 20, a rotary plate 24 fitted onto the thrust plate 22 and onto the hub portion thereof, the rotary plate 26 including a circumferential pattern of threaded bores 26 configured to be engaged by fasteners (e.g. fastening screws) FS26 which are provided for fastening the gripper 8 to the turntable 24. A closure plate 28 is fastened to the hub portion of the base plate 20 by fasteners (e.g. fastening screws FS28) to retain the turntable 24 in place and avoid axial separation thereof from the thrust plate 22 and the base plate 20. The pins 16(A), 16(B) are fixed to the turntable 24.

Similarly, the turntable assembly T2 of the carrier 6 comprises a base plate 30 with a protruding, internally threaded hub portion, a thrust plate 32 fitted onto the hub of the base plate 30 and having itself a hub portion fitted onto the hub portion of the base plate 30 and lying flush with the protruding hub portion of the base plate 30, a rotary plate 34 fitted onto the thrust plate 32 and onto the hub portion thereof, the rotary plate 36 including a circumferential pattern of threaded bores 36 configured to be engaged by fasteners (e.g. fastening screws) FS36 which are provided for fastening the gripper 10 to the turntable 34. A closure plate 38 is fastened to the hub portion of the base plate 30 by fasteners (e.g. fastening screws FS38) to retain the turntable 34 in place and avoid axial separation thereof from the thrust plate 32 and the base plate 30. The hubs 18(A), 18(B) are provided on the turntable 34, preferably integrally provided therewith.

The turntable assemblies T1, T2 including the grippers 8, 10 are mounted -respectively - onto a first and second upper roller units 40, 42, each including a staggered roller arrangement, wherein the staggering occurs along a direction parallel to the axes Z8, Z10. Specifically, the roller unit 40 is mounted onto the carrier 4 and includes a staggered pair of rollers R40, while the roller unit 42 is mounted onto the carrier 6 and includes a staggered pair of rollers R42 which partially overlaps the rollers R40 (see the two oppositely facing rollers in the mid section of the carrier unit 1).

On the opposite side of the carriers 4, 6 with respect to the roller units 40, 42 first and a second lower roller units 44, 46, each including a staggered roller arrangement, wherein the staggering occurs along a direction parallel to the axes Z8, Z10. Specifically, the roller unit 44 is mounted onto the carrier 4 and includes a staggered pair of rollers R44, while the roller unit 46 is mounted onto the carrier 6 and includes a staggered pair of rollers R46 which partially overlaps the rollers R44 (see the two oppositely facing rollers in the mid section of the carrier unit 1).

The lower roller units 44, 46 carry a respective counterweight 48, 50 to balance the weight of the turntable assemblies T1, T2 and the grippers 8, 10. Especially in the bends, the absence of counterweights on the carriers 4, 6 may result in a tip-over of the carrier unit due to the shifting of the overall centre of gravity up and out of the carriers 4, 6. In this sense, the counterweights 48, 50 reset the position of the centre of gravity to the carrier area: since engagement to the track(s) of the transfer path occurs in the area comprised between the roller units (which abut on the tracks and provide rolling support thereof, while the body of the carrier is magnetically engaged and driven by the tracks themselves), the centre of gravity has to remain in the engagement area as close as possible to the center thereof to avoid unbalancing inertial actions on the carriers.

In greater detail, each carrier 4, 6 has a first engagement portion E4A, E6A and a second engagement portion E4B, E6B opposite to the first engagement portion, whereby magnetic engagement can occur at either portion to accommodate for track changes along the transfer path (this is an inherent feature to the ACOPOSTrak^{®} carriers). As visible in figures 3 to 7, each of the first gripper 8 and the second gripper 10 is mounted on the respective carrier 4, 6 clear of the first engagement portion and the second engagement portion, and so are the turntable units T1, T2.

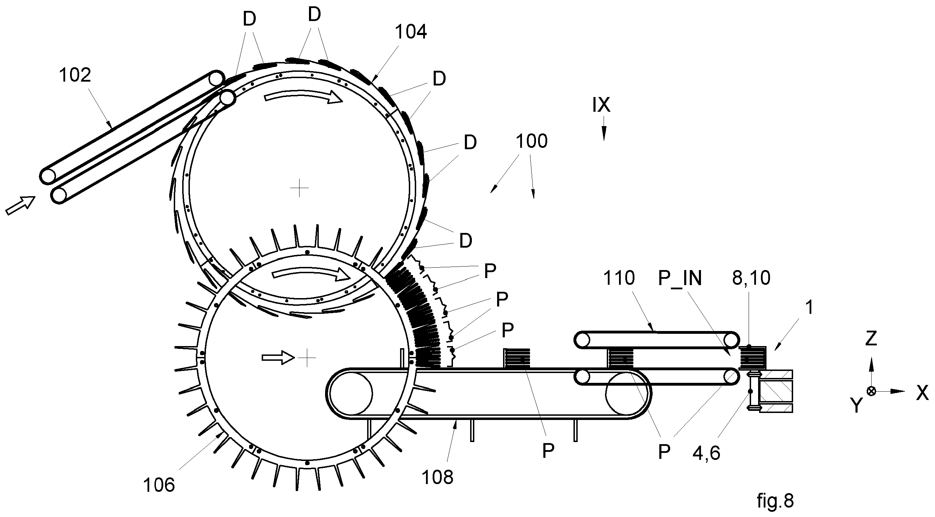

With reference to figures 8 to 11, operation of the carrier unit 1 will now be described. The description will be provided with reference - by way of example - to a preferred application wherein the product P is a stack of diapers (baby diapers or sanitary napkins)

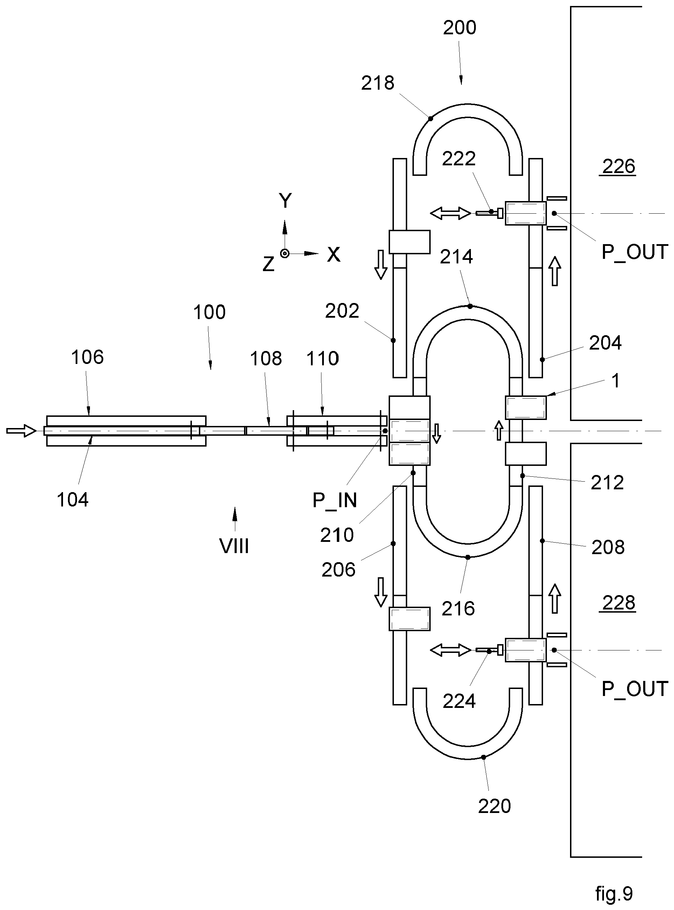

Carrier units 1 are configured to transfer products from a product input P_IN visible in figure 8 to one or more product output locations P_OUT visible in figure 9.

The product input P_IN (figure 8) corresponds to the location at which a carrier unit 1, so to say, takes delivery of a product P from a sorting station 100. The sorting station 100 comprises an input conveyor 102, a transfer wheel 104, a sorting wheel 106, a transfer conveyor 108 and an output conveyor 110. Any arrow reproduced on the elements above denotes a direction of motion (linear or rotational).

The input conveyor 102 receives individual individual diapers from upstream processing stages and delivers the same to the transfer wheel 104 (also known as "water wheel"). The transfer wheel 104 includes a plurality of vane shaped receptacles each configured for receiving and housing a diaper. The transfer wheel 104 then releases the diapers into sockets of the sorting wheel, which is configured to sort diapers into stacks defining the product P to be transferred by the carrier units 1. The sorting wheel 106 outputs the stacks of diapers onto the transfer conveyor 108, and the latter relays the products P to the output conveyor 110, which releases them to carrier units which sequentially line up at the product input P_IN.

Figure 9 shows the position of the sorting station 100 with respect to a transfer path 200 and the product output locations P_OUT. The transfer path 200 - here shown with a merely exemplary route pattern - includes an inner circle and an outer circle, the former mainly serving for buffering purposes. Each of the inner circle and the outer circle comprise straight portions and curved portions/bends.

As far as the straight portion, the outer circle comprises first and second straight portions 202, 204 on one side of the product input P_IN, and third and fourth straight portions 206, 208 on another, opposite, side of the product input P_IN. the inner circle comprises a fifth and a seventh straight portions 210, 212.

Inner bends/curved portions (U-shaped) 214, 216 connect the straight portions 210, 212 defining the inner circle, while outer bends/curved portions (U-shaped) 218, 220 connect the straight portions 202, 204 and 206, 208 respectively.

Product unloading devices 222, 224 are arranged at the product output locations P_OUT and are configured for unloading the products P from the carrier units 1 once negotiation of the transfer path from P_IN to P_OUT is completed. Product locations P_OUT correspond here to a first and a second packaging station 226, 228 whereat the products P relayed by the carrier units 1 are released by the unloading devices 222, 224.

The carrier units 1 are configured for negotiating the transfer path 200 - any of the portions thereof - by magnetic engagement and driving. Of course, other driving systems and engagement systems (e.g. mechanical driving and engagement systems) may be envisaged. The paired carriers 4, 6 are driven in paired arrangement by the driving system itself both in case of electromagnetic driving such as in the ACOPOSTrak^{®} flexible carriers, and in case of mechanical driving. Driving in paired arrangement also implies that the relative distance between the carriers 4, 6 is kept constant throughout the transfer path by the driving system itself. With electromagnetically driven carriers 4, 6 the inter-carrier distance can be adjusted by the driving system and kept constant, and can also be adjusted during travel, if required (for instance, when slowing down along rectilinear portions in the final approach to a product output location P_OUT).

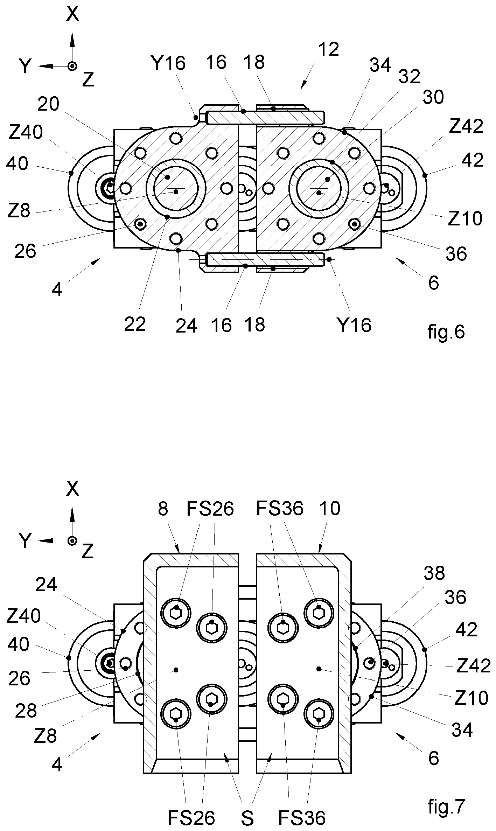

The engagement condition is visible in figure 11, wherein the contact between the rollers R40-R46 on the guides of the transfer path 200 is also visible. Figure 10 in combination with figures 3 and 7 shows the operation and the effect of the angle control device 12.

In prior art systems such as those in figures 1 and 2 the rigid connection between the grippers and the respective carriers results in the gripper following the carrier motion when the latter negotiates a bend. As a result, the gripper tend to "open up" and diverge because of the negotiation of a bend such as that visible in figures 1 and 2. In other words, the socket geometry is completely disrupted, as the relative position between the gripper changes during negotiation of the bend.

Thanks to the angle control device 12, the relative position between the grippers 8, 10 is continuously controlled during negotiation of a bend to maintain the predetermined, pre-set socket geometry (i.e the geometry of the socket S). More in detail, the angle control device 12 adjusts the angle between the gripper 8, 10 and the carrier 4, 6 to maintain a constant socket geometry: this is done through the sliding coupling of the two grippers through the pin-hub pairs 16(A)-18(A) and 16(B)-18(B). as said, the sole available degree of freedom for the grippers 8, 10 is the relative motion in the direction Y16, but the relative rotation, as well as any other type of relative displacement, is prevented from the double constraint provided by the pin-hub engagement (the same can be obtained by a single pin-hub engagement, only provided as a prismatic guide).

Once the desired socket geometry - for example the socket width determined by the distance between walls 8S-10S - is set by fixing the distance between the carriers 4, 6 (this can be done as a standard features on transfer system using flexible carriers such as the ACOPOSTrak^{®} carriers, as well as on fully mechanical systems), the socket geometry is maintained thanks to the sliding coupling between the grippers 8, 10 which automatically adjusts the gripper-carrier angle: as the grippers 8, 10 cannot rotate relative to one another due to the sliding coupling 16-18 (16(A)-18(A) and 16(B)-18(B)), the carriers 4, 6 rotate relative to the grippers 8, 10 (better put, they rotate "under" the grippers 8, 10) owing to the turntable units T1, T2, so that the carriers can adapt to and negotiate the transfer path 200 while the grippers are bound to one another by the sliding coupling. The angle between the grippers remains constant and equal to zero degrees, and so does the geometry of the socket S as the relative distance of the grippers 8, 10, hence of the inner side walls 8S, 10S, does not vary, while the angles A8 and A10 are continuously adjusted as a function of the radius of curvature, i.e. based on the features of the transfer path that are negotiated by the carrier unit 1. The angle control device 12 determines the amount of the angles A8 and A10 based on the preset geometry of the socket S and the radius of curvature. Such angles constitute sort of mechanical compensation between two different boundary (and driving) conditions: the shape of the transfer path (radius of curvature) and the geometry of the socket S.

Note also that angles A8 and A10 can instantaneously be different: the radius of curvature along the bends 214-220 may vary through the bend, whereby the leading carrier 4 or 6 may momentarily be located at a section of the bend with a different radius of curvature than the trailing carrier 6 or 4.

Maintaining a predetermined socket geometry also includes maintaining a predetermined socket dimension that varies according to a predetermined law or pattern, while maintaining the angle between the grippers 8, 10 at a zero value. For instance, while keeping the side walls 8S, 10S parallel to one another thanks to the angle control device 12, the inter-gripper distance along the direction Y may be varied according to a predetermined pattern (this, as indicated in the foregoing, can be easily achieved by electromagnetically driven carriers 4, 6) while the angles A8 and A10 are continuously adjusted (in either direction) to accommodate - in combination - the mechanical constraint set up by the angle control device 12, the radius of curvature of the transfer path and the inter-gripper distance.

Additionally, in alternative embodiments the angle control device 12 may not be based on a mechanical coupling, but is may rather comprise a first drive unit configured for controlling the first angle A8 and a second drive unit configured for controlling the second angle A10. The first drive unit and the second drive unit may comprise an electric motor configured for driving the turntable unit T1, T2. The drive units may be controlled by a transfer system control unit which, based on the preset geometry of the socket S and the position of the carrier 4, 6 along the transfer path 200 (the position is associated i.a. to a radius of curvature) determines the required value of the angles A8 and A10 to maintain the desired geometry of the socket S, thereby obtaining the same effect on the geometry of the socket S as the sliding coupling between pins 16 and hubs 18.

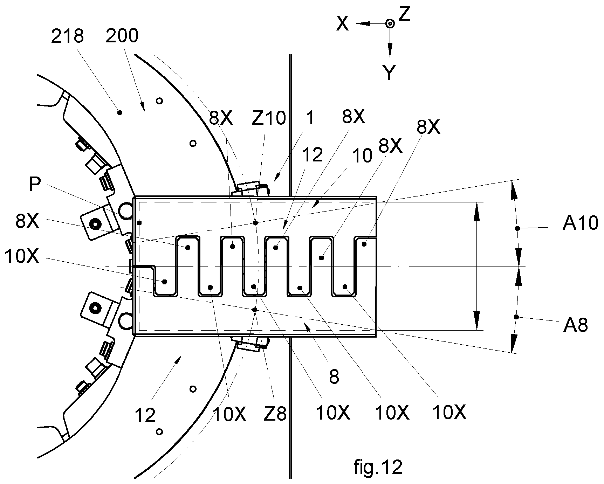

With reference to figure 12, in embodiments of the invention the shape of the grippers 8, 10 is modified to incorporate comb-like extensions 8X, 10X of the top walls 8T, 10T and/or the bottom walls 8B, 10B (the "and" alternative is preferred) and are configured to interdigitate to a variable extent - depending on the socket geometry - with the comb-like extensions of the other gripper as shown in figure 12. More in general, the comb-like extensions may be provided on the top walls 8T, 10T, or the bottom walls 8B, 10B. The comb-like extensions 8X, 10X may be provided on any of these pairs alone or in combination with the others.

These specific embodiments provide at least two key technical advantages, namely:
- first and foremost, the arrays of comb-like extensions 8X, 10X embody the angle control device 12, as the interdigitation of the extensions 8X, 10X allows the same degrees of freedom in the relative motion between the grippers 8, 10 as the pins 16 sliding through the hubs 18. Note in this respect that the only relative motion allowed for the grippers 8, 10 by the extensions 8X, 10X is a sliding motion along a direction which is overall parallel to the axes Y16 of the pins 16. This simplifies the construction of the carriers 4, 6 as the structural and geometric features required, for instance, to implement the pin-hub pairs 16, 18 or to accommodate the drive units can be actually dispensed with, thereby relying on the very grippers to provide the very angle control device 12 therefor;
- second, the extensions 8X, 10X globally bestow the socket S with full enclosure features regardless of the socket geometry. In other words, the interdigitation between the extensions 8X, 10X prevents the formation of a continuous gap (of course net of the operational plays) between the grippers 8, 10 throughout all the socket geometry range allowed by the length of the extensions 8X, 10X in the sliding direction. Maintenance of the geometry of the socket S is therefore achieved throughout all of the transfer path 200, whereby multiple transfer patterns can be implemented therethrough without any of the technical problems affecting the prior art. The transfer system may operate at maximum speed even in the bends because the interference condition determined by the geometry of the socket S will not change from a straight portion to a curved/bend portion thanks to the angle control device 12.

## Claims

1. A carrier unit (1) for a transfer system comprising a transfer path (200), the carrier unit (1) comprising a first carrier (4) and a second carrier (6), the first and the second carriers (4, 6) being engageable to the transfer path (200) and configured for negotiating the transfer path (200), wherein the first carrier (4) is coupled to a first gripper (8) configured for cooperating with a second gripper (10) coupled to the second carrier (6) to define a socket (S) for holding one or more products (P), the socket (S) having a socket geometry determined by a relative position of the first gripper (8) with respect to the second gripper (10), and wherein:
- the first gripper (8) is rotatably coupled to the first carrier (4) around a respective first axis of rotation (Z8), whereby the relative position of the first gripper (8) with respect to the first carrier (4) is defined by a first angle (A8) between the first gripper (8) and the first carrier (4) around the first axis of rotation (Z8),
- the second gripper (10) is rotatably coupled to the second carrier (6) around a respective second axis of rotation (Z10), whereby the relative position of the second gripper (10) with respect to the second carrier (6) is defined by a second angle (A10) between the second gripper (8) and the second carrier (6) around the second axis of rotation (Z10),
- the carrier unit (1) comprises an angle control device (12) configured for adjusting the first angle (A8) and the second angle (A10) to maintain a predetermined socket geometry upon negotiation of the transfer path (200) by the carrier unit (1),
the carrier unit being **characterized in that** the angle control device (12) comprises arrays of comb-like extensions (8X, 10X) of top walls (8T, 10T) and/or bottom walls (8B, 10B) of the grippers (8, 10), the comb-like extensions (8X, 10X) of one gripper (8, 10) being configured to interdigitate with the comb-like extensions of the other gripper (10, 8).

2. The carrier unit (1) of claim 1, wherein the socket geometry includes a socket width defined as the distance between facing inner walls (8S, 10S) of the first gripper (8) and the second gripper (10) along a direction transverse to the first axis of rotation (Z8) and the second axis of rotation (X10), and extending across them.

3. The carrier unit (1) of Claim 2, wherein the predetermined socket geometry comprises a predetermined socket width.

4. A transfer system comprising a transfer path (200) and one or more carrier units (1) according to any of the previous claims, each of the carrier units (1) being configured to engage and negotiate portions of the transfer path (200) to transfer the products (P) carried thereby along the transfer path (200).

## Patentansprüche

1. Trägereinheit (1) für ein Transfersystem, umfassend einen Transferweg (200), wobei die Trägereinheit (1) einen ersten Träger (4) und einen zweiten Träger (6) umfasst, wobei der erste und der zweite Träger (4, 6) an den Transferweg (200) anlegbar und zum Durchfahren des Transferwegs (200) ausgebildet sind,
wobei der erste Träger (4) an einen ersten Greifer (8) gekoppelt ist, der zum Zusammenwirken mit einem zweiten Greifer (10) ausgebildet ist, der mit dem zweiten Träger (6) gekoppelt ist, um eine Aufnahme (S) zum Halten eines oder mehrerer Produkte (P) zu definieren, wobei die Aufnahme (S) eine Aufnahmegeometrie aufweist, die durch eine relative Position des ersten Greifers (8) in Bezug zum zweiten Greifer (10) bestimmt ist, und wobei:
- der erste Greifer (8) drehbar mit dem ersten Träger (4) um eine jeweilige erste Drehachse (Z8) gekoppelt ist, wobei die relative Position des ersten Greifers (8) in Bezug zum ersten Träger (4) durch einen ersten Winkel (A8) zwischen dem ersten Greifer (8) und dem ersten Träger (4) um die erste Drehachse (Z8) definiert ist,
- der zweite Greifer (10) drehbar mit dem zweiten Träger (6) um eine jeweilige zweite Drehachse (Z10) gekoppelt ist, wobei die relative Position des zweiten Greifers (10) in Bezug zum zweiten Träger (6) durch einen zweiten Winkel (A10) zwischen dem zweiten Greifer (8) und dem zweiten Träger (6) um die zweite Drehachse (Z10) definiert ist,
- die Trägereinheit (1) eine Winkelregelungsvorrichtung (12) umfasst, die zum Anpassen des ersten Winkels (A8) und des zweiten Winkels (A10) ausgebildet ist, um eine vorbestimmte Aufnahmegeometrie beim Durchfahren des Transferwegs (200) durch die Trägereinheit (1) aufrechtzuerhalten,
wobei die Trägereinheit **dadurch gekennzeichnet ist, dass** die Winkelregelungsvorrichtung (12) Anordnungen von kammartigen Erstreckungen (8X, 10X) von oberen Wänden (8T, 10T) und/oder unteren Wänden (8B, 10B) der Greifer (8, 10) umfasst, wobei die kammartigen Erstreckungen (8X, 10X) eines Greifers (8, 10) ausgebildet sind, um mit den kammartigen Erstreckungen des anderen Greifers (10, 8) ineinander zu greifen.

2. Trägereinheit (1) nach Anspruch 1, wobei die Aufnahmegeometrie eine Aufnahmebreite einschließt, die als der Abstand zwischen den einander zugewandten Innenwänden (8S, 10S) des ersten Greifers (8) und des zweiten Greifers (10) entlang einer Richtung quer zur ersten Drehachse (Z8) und zur zweiten Drehachse (X10) definiert ist und sich über diese erstreckt.

3. Trägereinheit (1) nach Anspruch 2, wobei die vorbestimmte Aufnahmegeometrie eine vorbestimmte Aufnahmebreite umfasst.

4. Transfersystem, umfassend einen Transferweg (200) und eine oder mehrere Trägereinheiten (1) entsprechend einem der vorstehenden Ansprüche, wobei jede der Trägereinheiten (1) zum Anlegen an Abschnitte des Transferwegs (200) und zum Durchlaufen dieser ausgebildet ist, um die dadurch getragenen Produkte (P) entlang des Transferwegs (200) zu transferieren.

## Revendications

1. Unité de support (1) pour un système de transfert comprenant un chemin de transfert (200), l'unité de support (1) comprenant un premier support (4) et un deuxième support (6), les premier et deuxième supports (4, 6) pouvant venir en prise sur le chemin de transfert (200) et étant configurés pour négocier le chemin de transfert (200),
dans laquelle le premier support (4) est couplé à un premier préhenseur (8) configuré pour coopérer avec un deuxième préhenseur (10) couplé au deuxième support (6) pour définir une tubulure (S) pour maintenir un ou plusieurs produits (P), la tubulure (S) présentant une géométrie de tubulure déterminée par une position relative du premier préhenseur (8) par rapport au deuxième préhenseur (10), et dans laquelle :
- le premier préhenseur (8) est couplé en rotation au premier support (4) autour d'un premier axe de rotation (Z8) respectif, de telle sorte que la position relative du premier préhenseur (8) par rapport au premier support (4) soit définie par un premier angle (A8) entre le premier préhenseur (8) et le premier support (4) autour du premier axe de rotation (Z8),
- le deuxième préhenseur (10) est couplé en rotation au deuxième support (6) autour d'un deuxième axe de rotation (Z10) respectif, de telle sorte que la position relative du deuxième préhenseur (10) par rapport au deuxième support (6) soit définie par un deuxième angle (A10) entre le deuxième préhenseur (8) et le deuxième support (6) autour du deuxième axe de rotation (Z10),
- l'unité de support (1) comprend un dispositif de commande d'angle (12) configuré pour régler le premier angle (A8) et le deuxième angle (A10) pour maintenir une géométrie de tubulure prédéterminée lors de la négociation du chemin de transfert (200) par l'unité de support (1), l'unité de support étant **caractérisée en ce que** le dispositif de commande d'angle (12) comprend des matrices d'extensions en forme de peigne (8X, 10X) de parois supérieures (8T, 10T) et/ou de parois inférieures (8B, 10B) des préhenseurs (8, 10), les extensions en forme de peigne (8X, 10X) d'un préhenseur (8, 10) étant configurées pour s'interdigiter avec les extensions en forme de peigne de l'autre préhenseur (10, 8).

2. Unité de support (1) selon la revendication 1, dans laquelle la géométrie de tubulure inclut une largeur de tubulure définie comme la distance entre des parois internes opposées (8S, 10S) du premier préhenseur (8) et du deuxième préhenseur (10) le long d'une direction transversale au premier axe de rotation (Z8) et au deuxième axe de rotation (X10), et s'étendant à travers eux.

3. Unité de support (1) selon la revendication 2, dans laquelle la géométrie de tubulure prédéterminée comprend une largeur de tubulure prédéterminée.

4. Système de transfert comprenant un chemin de transfert (200) et une ou plusieurs unités de support (1) selon l'une quelconque des revendications précédentes, chacune des unités de support (1) étant configurée pour venir en prise et négocier des portions du chemin de transfert (200) pour transférer les produits (P) ainsi transportés le long du chemin de transfert (200).
